# DEMANDE DE BREVET EUROPEEN

(11) **EP 0 688 874 A2**
(43) Date de publication de la demande: **27.12.1995**
(21) Numéro de dépôt: 95112386.8
(22) Date de dépôt: 17.08.1990
(51) Int. Cl.: C12Q 1/70, C07K 14/03, C12N 7/04, C12N 15/38, C12N 1/21, C12Q 1/68

(54) **Fragments d'acides nucléiques et réactifs de diagnostic dérivés du génome de HHV6/SIE et procédés de diagnostic des infections à HHV6**

(30) Priorité: 18.08.1989 FR 8911016
(62) Demande divisionnaire de: 90912921.5
(71) Demandeur: INSTITUT PASTEUR, F-75724 Paris Cédex 15 (FR); UNIVERSITE PARIS VI, F-75005 Paris (FR)
(72) Inventeur: Collandre, Hélène, F-75018 Paris (FR); Montagnier, Luc, F-92350 Le Plessis-Robinson (FR); Gut, Henri, F-75016 Paris (FR); Bechet, Jean Marie, F-75007 Paris (FR)
(74) Mandataire: Vialle-Presles, Marie José

(57) **Abrégé**

L'invention est relative à des fragments l'acide nucléique dérivés du génome du virus HHV6, à des vecteurs contenant lesdits fragments, ainsi qu'à leur utilisation dans le diagnostic des infections impliquant ce virus.

## Description

La présente invention est relative à des fragments d'acides nucléiques dérivés du génome du virus HHV6, à des vecteurs contenant lesdits fragments ainsi qu'à leur utilisation dans le diagnostic des infections impliquant ce virus.

Les virus HHV6, qui sont des virus à ADN double brin, classés dans la famille des Herpes virus, ont été isolés à partir de lymphocytes de patients atteints de SIDA ou présentant des désordres lymphoprolifératifs. Ces virus sont également considérés comme étant l'agent causal de l'exanthème subit.

Différentes souches de ces virus sont connues ; la souche HBLV, qui infecte spécifiquement les lymphocytes B, a été décrite par SALAHUDIN et al. (Science 234, 396, 1986), et dans la demande PCT WO 88/01387 ; deux autres souches ont été décrites par les Inventeurs : la souche 39 TAN (AGUT et al., Res. Virol. 140, 23, 1989) et la souche SIE, qui infecte les lymphocytes T (AGUT et al., Lancet i, 712, 1988).

Le génome de la souche SIE, d'une taille d'environ 160 kb est constitué d'ADN double brin sous forme essentiellement linéaire ; sa teneur en GC est comprise entre 38 et 40%, et il possède des séquences terminales répétitives.

La présente invention a pour but de fournir des réactifs d'acide nucléique permettant le diagnostic spécifique des infections à HHV6/SIE, et également des réactifs d'acide nucléique permettant le diagnostic général des infections impliquant une quelconque souche d'HHV6. Dans ce but, les Inventeurs ont sélectionné, cloné et caractérisé un fragment du génome viral de HHV6/SIE.

La présente invention a pour objet un fragment de l'ADN génomique du virus HHV6/SIE, caractérisé en ce qu'il est obtenu par digestion de l'ADN génomique du virus HHV6/SIE avec l'enzyme Bam HI, en ce que sa taille est de 5 kb et en ce qu'il contient un site de restriction EcoRI à 1,8 kb de l'une des ses extrémités.

La présente invention englobe également des séquences nucléotidiques synthétiques, semi-synthétiques ou obtenues par génie génétique, dérivées d'un fragment d'ADN tel que défini plus haut, qu'il s'agisse de séquences d'ADN double brin contenant tout ou partie de la séquence dudit fragment, ou d'ADN ou d'ARN simple brin complémentaires de tout ou partie de l'un ou l'autre brin dudit fragment.

Un mode de réalisation préféré de la présente invention englobe des séquences d'ADN recombinant, contenant tout ou partie de la séquence du fragment du génome de HHV6/SIE conforme à l'invention, telle que en particulier :
- le plasmide pHC-147, qui résulte de l'insertion du fragment d'ADN de 5 kb défini plus haut dans le plasmide PTZ 19R.

La présente invention a également pour objet des clones de cellules eucaryotes ou procaryotes transformées, contenant au moins une séquence d'ADN recombinant telle que définie plus haut.

Un clone de la souche DH5 d'Escherichia coli, transformé par le plasmide pHC-147 mentionné ci-dessus, a fait l'objet d'un dépôt auprès de la Collection Nationale de Cultures de Microorganismes (CNCM), le 19 Juillet 1989, sous le numéro de dépôt I-896.

La présente invention a également pour objet des réactifs de diagnostic, caractérisés en ce qu'ils contiennent au moins une des séquences nucléotidiques dérivées du fragment Bam HI de 5 kb du génome du virus HHV6/SIE conforme à l'invention, telles qu'elles ont été définies précédemment, associée à au moins un moyen de détection approprié.

Lesdits réactifs peuvent être utilisés dans un très grand nombre de techniques de diagnostic basées sur la détection d'acides nucléiques par hybridation et/ou amplification .

La présente invention a en conséquence pour objet un procédé de diagnostic permettant soit la détection du virus HHV6/SIE, soit de façon plus générale, la détection du virus HHV6 dans un échantillon biologique, caractérisé en ce qu'il comprend une étape dans laquelle l'on met en contact l'échantillon biologique avec un réactif conforme à l'invention, et une étape dans laquelle on détecte une interaction spécifique entre ledit réactif et une ou plusieurs séquences de l'ADN dudit virus éventuellement présent dans l'échantillon biologique.

Selon un mode de mise en oeuvre préféré de ce procédé, la détection est effectuée par la méthode d'amplification PCR (amplification en chaîne par la Taq polymérase) et on utilise comme amorces et éventuellement comme sondes des séquences oligonucléotidiques dérivées du fragment Bam HI de 5 kb défini plus haut.

La présente invention a en outre pour objet un kit pour le diagnostic des infections HHV6 et/ou HHV6/SIE, caractérisé en ce qu'il comprend au moins un réactif conforme à l'invention, associé à des moyens de mise en oeuvre d'un procédé tel que décrit plus haut.

La présente invention sera mieux comprise à l'aide du complément de description qui va suivre, qui se réfère à des exemples de préparation des réactifs conformes à l'invention et de leur utilisation pour la détection de HHV6.

### EXEMPLE : PREPARATION DES FRAGMENTS ET DES MOLECULES D'ACIDE NUCLEIQUE CONFORMES A L'INVENTION

### A) ISOLEMENT ET PROPAGATION DE LA SOUCHE HHV6/SIE

La souche HHV6/SIE a été isolée à partir des lymphocytes d'une patiente atteinte d'une leucémie à HTLV-1 et séropositive pour HIV-2.

Le virus est propagé sur lymphocytes de sang périphérique. L'isolement et la propagation de cette souche sont décrites dans la publication de AGUT et al. (Lancet i, 712, 1988).

### B) OBTENTION ET CARACTERISATION DES FRAGMENTS ET DES MOLECULES D'ACIDE NUCLEIQUE CONFORMES A L'INVENTION

### Obtention et clonage du fragment Bam HI de 5 kb

L'ADN extrait des cellules PBL (lymphocytes de sang périphérique) infectées, est totalement digéré par l'enzyme Bam HI.

Les fragments résultants de la digestion sont séparés sur gradient de saccharose 10-30%.

Les fragments de taille inférieure à 15 kb sont insérés au site Bam HI du plasmide multifonctionnel PTZ 19R, et introduits dans les bactéries E.coli DH5.

La banque génomique ainsi obtenue est criblée par de l'ADN viral isolé du virion purifié sur gradient de saccharose 10-60% et marqué au CTP³²P.

Un clone recombinant contenant le plasmide pHC 147 qui comporte un insert de 5 kb de l'ADN génomique de HHV6/SIE. Ce clone a été déposé le 19 Juillet 1989 auprès de la CNCM sous le numéro d'accès I-896 a été ainsi sélectionne.

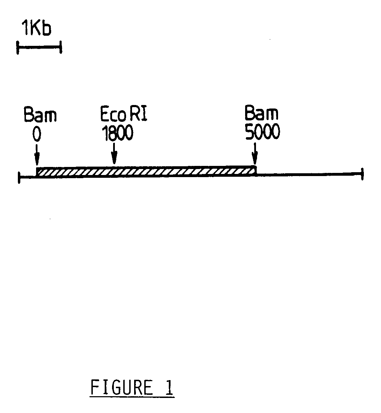

La Figure 1 représente la carte de restriction du plasmide pHC 147.

La zone hachurée indique l'emplacement de l'insert de 5 kb. Cet insert porte un site de restriction EcoRI à 1,8 kb de l'une de ses extrémités. Il ne contient aucun site pour les enzymes Sma I, Sal I, Sac I, Kpm I, Xba I.

## Revendications

1. Fragment de l'ADN génomique du virus HHV6/SIE, caractérisé en ce qu'il est susceptible d'être obtenu par digestion avec l'enzyme Bam HI, et en ce que sa taille est d'environ 5 kb et en ce qu'il contient un site de restriction EcoRI à 1,8 kb de l'une de ses extrémités.

2. Séquence nucléotidique caractérisée en ce qu'elle comprend tout ou partie d'un fragment d'ADN selon la revendication 1, ou en ce qu'elle est complémentaire de tout ou partie dudit fragment.

3. Séquence nucléotidique selon la revendication 2, caractérisée en ce qu'elle obtenue par recombinaison.

4. Séquence d'ADN recombinant selon la revendication 3, caractérisée en ce qu'elle est constituée par le plasmide pHC-147, lequel plasmide est obtenu par l'insertion dans le plasmide PTZ 19R d'un fragment d'ADN selon la revendication 1.

5. Clone de cellules eucaryotes ou procaryotes transformées, caractérisé en ce qu'il contient au moins une séquence d'ADN recombinant selon la revendication 1.

6. Clone selon la Revendication 5, déposé à la Collection Nationale de Cultures de Microorganismes (CNCM) sous le N°I-896 et caractérisé en ce qu'il est constitué par des cellules bactériennes de la souche *E.coli* DH5 transformées par le plasmide pHC-147.

7. Réactif de diagnostic, caractérisé en ce qu'il contient au moins une séquence nucléotidique selon l'une quelconque des revendications 2 à 4 associée à au moins un moyen de détection approprié.

8. Procédé de diagnostic de la présence du virus HHV6/SIE et/ou du virus HHV6 dans un échantillon biologique, caractérisé en ce qu'il comprend une étape dans laquelle l'on met en contact l'échantillon biologique avec un réactif selon la revendication 7, et une étape dans laquelle on détecte une interaction spécifique entre ledit réactif et une ou plusieurs séquences de l'ADN dudit virus éventuellement présent dans l'échantillon biologique.

9. Procédé selon la revendication 8, comprenant une étape d'amplification, effectuée par la méthode d'amplification PCR, caractérisé en ce qu'on utilise comme amorces et éventuellement comme sondes des séquences oligonucléotidiques dérivées de fragments d'ADN selon la revendication 1.

10. Kit pour le diagnostic des infections HHV6 et/ou HHV6/SIE, caractérisé en ce qu'il comprend au moins un réactif selon la revendication 7, associé à des moyens de mise en oeuvre d'un procédé selon une quelconque des revendications 8 ou 9.
